# EUROPEAN PATENT APPLICATION

(11) **EP 4 810 015 A1**
(43) Date of publication of application: **23.09.2026**
(21) Application number: 24891861.7
(22) Date of filing: 13.11.2024
(51) Int. Cl.: A61K 9/00, A61M 37/00

(54) **MICRONEEDLE STRUCTURE AND COMPOSITION THEREOF**

(30) Priority: 13.11.2023 KR 20230156002
(71) Applicant: CGBIO Co.,Ltd., Seoul 04349 (KR)
(72) Inventor: MIN, Seon-hong, Incheon 21982 (KR); SEO, Jihye, Hwaseong-si Gyeonggi-do 18488 (KR); PARK, Hyuna, Seongnam-si Gyeonggi-do 13356 (KR); HA, Hyeran, Ansan-si Gyeonggi-do 15541 (KR); KIM, Bongsoo, Hwaseong-si Gyeonggi-do 18478 (KR)
(74) Representative: Viering, Jentschura & Partner mbB Patent- und Rechtsanwälte
(86) International application number: PCT/KR2024/096509
(87) International publication number: WO 2025/105915

(57) **Abstract**

The present invention relates to a microneedle structure and a composition thereof, the microneedle structure comprising a biodegradable composition containing a medicinal substance and hyaluronidase so that the medicinal substance can be effectively delivered percutaneously, locally or throughout the body, through the dermal layers without injury to the skin.

## Description

### Technical Field

The present disclosure relates to a microneedle structure and a composition thereof.

### Background Art

Microneedles are methods widely used as drug delivery systems. They have advantages capable of minimizing pain and improving therapeutic delivery speed through a mechanism that directly guides drugs into the subcutaneous region. Furthermore, localized drug injection allows for effective drug deliver to the desired area only.

However, dissolvable microneedle structures have been caused a problem of having limitations in drug loading amount due to their limited drug application capacity so that various attempts have been made to solve this problem, but the technology for quantitative drug delivery has not been solved.

Meanwhile, most transdermal microneedles are made of water-soluble polymers, resulting in minimal skin diffusion, and even for drugs with high skin diffusion, it is difficult to release the drug transdermally if the solubility of the microneedle itself is low. Furthermore, in order to penetrate the skin, the strength of the needle must be high, but substances that increase the strength of the needle have difficulty dissolving in skin moisture, which can cause wounds, and there is a problem that the drug diffusion rate is low, so it must be injected at a higher concentration than an injection.

Therefore, there is an urgent need to develop a form that can effectively deliver drugs transdermally without causing wounds in the skin.

### DISCLOSURE

### Technical Problem

An object of the present disclosure that has been derived solve the above-described problems is to provide a microneedle structure that can effectively deliver a drug locally or systemically through the dermis layer by percutaneously penetrating the skin without causing wounds in the skin, and a composition thereof.

### Technical Solution

To achieve the above objective, a microneedle structure composition according to one embodiment of the present disclosure includes a drug and a biodegradable composition, wherein the biodegradable composition includes hyaluronidase.

Here, hyaluronidase may be contained in an amount of 10 to 1,500 IU per sheet.

A microneedle structure for drug delivery according to another embodiment of the present disclosure includes a substrate and one or more microneedles disposed on the substrate, wherein the microneedles contain a drug and a biodegradable composition, and the biodegradable composition includes hyaluronidase.

At this time, the microneedles may increase the transdermal absorption rate of the drug compared to microneedles that do not contain hyaluronidase.

Further, the substrate may have a thickness of 10 to 300 µm.

Further, the microneedles may have a length of 100 to 1,500 µm.

### Advantageous Effects

According to one embodiment of the present disclosure, a microneedle structure and a composition thereof can effectively deliver the drug locally or systemically to the body through the dermis layer without causing skin wounds by percutaneous penetration by including a biodegradable composition containing a drug and hyaluronidase.

In addition, side effects can be minimized and efficacy maximized by adjusting the drug release rate or making it possible to deliver the drug in an appropriate amount as needed to the target area.

### Brief Description of Drawings

FIG. 1 is a schematic cross-sectional view of a microneedle structure according to an embodiment of the present disclosure.
FIG. 2 is a diagram showing the results of Experimental Example 1.
FIGS. 3A and 3B are optical micrographs showing results according to Experimental Example 1.

### Mode for Invention

Hereinafter, the present disclosure will be described in detail as exemplary embodiments thereof with reference to the attached drawings. However, the following exemplary embodiments are provided as illustrative examples of the present disclosure, and if a detailed description of a technology or configuration well known to those skilled in the art is deemed to unnecessarily obscure the gist of the present disclosure, such detailed description may be omitted, and the present disclosure is not limited thereby. The present disclosure is capable of various modifications and applications within the scope of the description of claims described later and equivalents interpreted therefrom.

Furthermore, the terminologies used in the present specification are terms used to appropriately express preferred embodiments of the present disclosure, and these may vary depending on the intent of the user or operator or the practices of the field to which the present disclosure pertains. Therefore, definitions of the present terms should be based on the overall content of the present specification. Throughout the specification, when a part is said to "includes" a component, it may mean that this does not exclude other components, but rather include other components, unless otherwise stated.

Throughout the present specification, in the term "%" that is used to indicate the concentration of a specific substance, "%" refers to solid/solid (w/w), "%" refers to solid/liquid (w/v), and "%" refers to liquid/liquid (v/v), unless otherwise specified.

In the present disclosure, after a microneedle structure pierces the skin, wound healing through epithelial cell migration and restoration of the ability to prevent penetration of external substances through restoration of the skin barrier occur within 2 to several hours depending on the size of the wound so that the drug absorption rate was improved by increasing the drug absorption rate of the structure using hyaluronidase in order to solve the problem of difficulty in continuous absorption of drugs. Hyaluronidase has been known to break down hyaluronic acid, which exists subcutaneously in the human body, thereby increasing drug permeability and speeding up drug diffusion. For example, when a sperm penetrates an egg, hyaluronidase is secreted from the sperm head to melt the surface surrounding the egg, and the sperm can penetrate into places where part of the membrane melts and is perporated.

The microneedle structure composition for drug delivery referred to in the present disclosure includes a drug and hyaluronidase, and may further include one or more selected from a biodegradable polymer, a saccharide, purified water, and an additive.

The biodegradable composition referred to in the present disclosure includes hyaluronidase, and may further include one or more selected from a biodegradable polymer, a saccharide, and an additive.

The microneedle structure for drug delivery referred to in the present disclosure may include a state in which all or part of purified water has evaporated from the microneedle structure composition for drug delivery.

The single sheet referred to in the present disclosure may be an entire microneedle structure composition for drug delivery or an entire microneedle structure for drug delivery.

Hereinafter, a microneedle structure composition according to one embodiment of the present disclosure and a microneedle structure manufactured therefrom will be described in detail.

The microneedle structure composition includes a drug and a biodegradable composition.

The drug may refer to an ingredient with efficacy, and may be, for example, one or more selected from among chemical pharmaceuticals including at least one of pentoxifylline, bimatoprost, latanoprost, travoprost, statin, resveratrol, deoxycholic acid, lithocholic acid, isoproterenol, prostaglandins, polynucleotides, polydeoxynucleotides, lidocaine, donepezil, and salicylic acid, growth factors including at least one of EGF, TGF, and BMP, peptides, and proteins. Such drugs may be included in an amount of 1 to 21 parts by weight relative to the total parts by weight of the microneedle structure composition.

The biodegradable composition may include hyaluronidase, hyaluronidase is added to facilitate drug penetration through the dermis layer into the muscle layer or capillaries, thereby enhancing drug action within the body, and the composition may contain 10 to 1500 international units (IU) per sheet. When hyaluronidase is included within the above concentration range, the transdermal absorption rate of the drug may be enhanced. In the present embodiment, side effects can be minimized and efficacy can be maximized by adjusting the drug release rate or making it possible to deliver an appropriate amount of the drug as needed to the target site through the content of hyaluronidase. Furthermore, the drug delivery efficiency can be improved, thereby improving dosage limits. Meanwhile, hyaluronidase included in the biodegradable composition of the present disclosure may vary in enzyme activity depending on the biodegradable composition or final product, but the amount added may be controlled to satisfy the above concentration range.

For example, the biodegradable composition may include one or more selected from a biodegradable polymer, a saccharide, and an additive. Such biodegradable composition may be included in an amount of 79 to 99 parts by weight based on the total parts by weight of the microneedle structure composition, and since inclusion of a large amount of the biodegradable composition may weaken the strength of the microneedle structure to lower skin permeability, it is desirable to increase drug delivery efficiency by satisfying the above-mentioned range and maintaining the strength of the microneedle.

The biodegradable polymer may include a polysaccharide including at least one of alginate, carrageenan, gelatin, carboxymethyl cellulose (CMC), and aloe polysaccharide (APS), one or more selected from polyvinyl pyrrolidone (PVP), polyvinyl alcohol (PVA), polyvinylacetate (PVAc), polyethylene glycol (PEG), polypropylene glycol (PPG), polyethylene oxide (PEO), polypropylene oxide (PPO), polylactic acid (PLA), polylactide (PL), polyglycolic acid (PGA), polycaprolactone (PCL), hydroxypropyl methylcellulose (HPMC), chondroitin sulfate, dextrin, dextran, dextran sulfate, ethylcellulose, collagen, agarose, cyclodextrin, heparosan, and sodium chondroitin sulfate, for example, one or more selected from alginate, carrageenan, gelatin, carboxymethyl cellulose (CMC), polyvinyl alcohol (PVA), poloxamer, polyvinyl pyrrolidone (PVP), hydroxypropyl methylcellulose (HPMC), and ethylcellulose, and specific examples thereof may include one or more selected from gelatin, carboxymethyl cellulose (CMC), polyvinyl alcohol (PVA), poloxamer, and polyvinyl pyrrolidone (PVP). These biodegradable polymers may be included in an amount of 50 to 99 parts by weight per 100 parts by weight of the biodegradable composition.

The biodegradable polymer may be included in an amount of 40 to 85 parts by weight with respect to the total parts by weight of the microneedle structure.

The saccharide is substances added to accelerate drug diffusion and may include one or more selected from mannitol, glucose, sucrose, maltose, sucrose, trehalose, and lactose. Such a saccharide may be included in an amount of 7.5 to 32 parts by weight with respect to 100 parts by weight of the biodegradable composition.

The saccharide may be included in an amount of 7 to 25 parts by weight with respect to the total parts by weight of the microneedle structure.

The additive may include one or more selected from a plasticizer, a surfactant, a preservative, and an antiinflammatory agent, such an additive may be included in a small amount as long as it does not affect the biodegradable composition, and the content thereof is not significantly limited.

One or more selected from ethylene glycol, propylene glycol, dipropylene glycol, butylene glycol, and glycerin may be used as the plasticizer.

One or more selected from polysorbate 80, polysorbate 20, polyoxyethylene (POE), and polyoxypropylene glycol (POP) may be used as the surfactant.

Hereinafter, a microneedle structure according to another embodiment of the present disclosure will be described in detail.

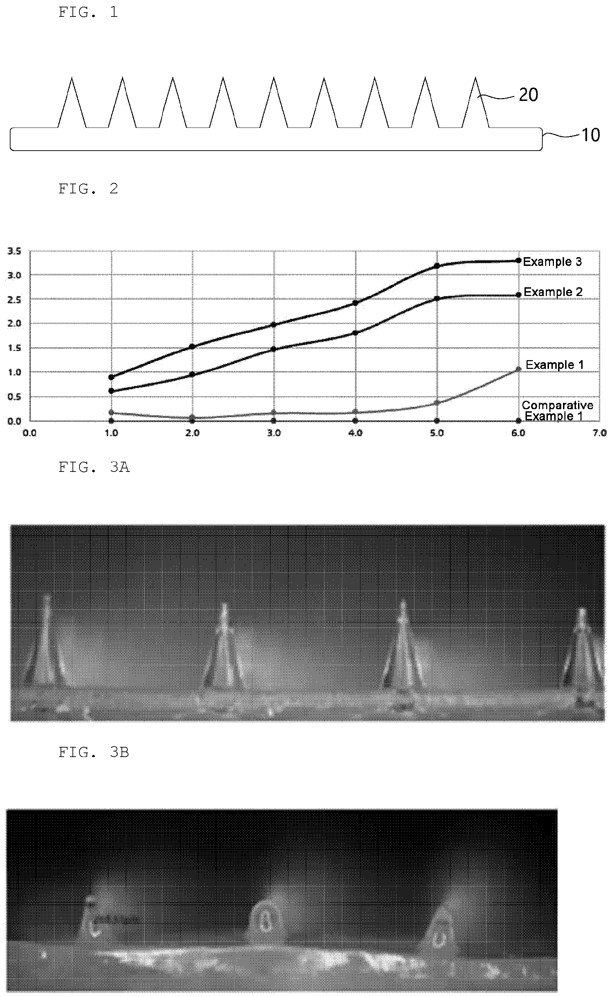

Referring to FIG. 1, a microneedle structure 100 may include a substrate 10 and microneedles 20.

The substrate 10 may be formed of a biodegradable material with certain hardness and strength that enable support of one or more microneedles 20. For example, the substrate 10 may include a biodegradable polymer, which has been described in detail above and will therefore be omitted. For example, the substrate 10 preferably has a thickness of 10 to 300 µm, for example, 100 to 200 µm, if the substrate has a thickness less than 10 µm, it may be too thin to easily bend or tear, making it difficult to protect the drug, and if the substrate has a thickness exceeding 300 µm, it may be difficult to adhere it well to curved areas when adhered to the skin.

Meanwhile, the substrate 10 may be formed integrally with the microneedles 20 using a mold, and may also be manufactured from the same components, but is not limited thereto. Furthermore, although not illustrated in the drawing, the substrate 10 may be formed of one or more layers, in which case adjacent layers may have different hardnesses and strengths. For example, the hardness and strength may decrease as the substrate approaches the microneedles 20. Therefore, this enables it to be applied to a wider range of body parts and applications.

The microneedles 20 may be disposed one or more times on the substrate 10 so that the microneedles 20 may be disposed at a predetermined interval, and unlike conventional injection needles, may penetrate the skin without causing trauma or pain. For example, the microneedles 20 are formed in a cone shape pointed in the opposite direction to the substrate 10, and preferably has a strength of 0.05 N or more, for example, 0.05 to 3.04 N, or another example, 0.05 to 1.0 N, while satisfying a length of 100 to 1500 µm so as to be able to deliver drugs to the muscle layer or capillaries by penetrating the stratum corneum of the skin without changing its shape. When the hardness and length range of the microneedles 20 described above are satisfied, the microneedles 20 can more accurately deliver drugs to the desired location in the body through the dermis layer by penetrating the skin without changing the microneedles' shape and without causing wounds in the skin.

The microneedles 20 may include a biodegradable composition and a drug, and the biodegradable composition may include one or more selected from a biodegradable polymer, a saccharide, and an additive. The composition is omitted as it overlaps with the description above. The microneedles 20 of the present embodiment may increase the transdermal absorption rate of the drug compared to microneedles that do not contain hyaluronidase.

Meanwhile, although not shown in the drawing, the microneedles 20 may be formed as a single layer or multiple layers, and when the microneedles 20 are formed as multiple layers, the compositions of the respective layers may be the same as or different from.

Hereinafter, the present disclosure will be further decribed using Examples. It will be apparent to those skilled in the art that these Examples are intended solely to describe the present disclosure more specifically and that the scope of the present disclosure is not limited thereby.

### Examples

### Examples 1 to 3. Manufacturing of Microneedle Structures

Biodegradable compositions according to Examples of the present disclosure were added to 91.8 g of purified water and stirred, and specifically, 4.5 g of biodegradable polymers were added and stirred at 500 rpm for 40 minutes, and then, 2 g of mannitol was added and stirred further. Thereafter, 2 g of sodium polynucleotide (PN) was added and stirred at 700 rpm for 15 minutes, and hyaluronidase was added at the amounts listed in Table 1, sufficiently dissolved, degassed, and then dispensed into a microneedle mold. This was then dried in a pressurized chamber to manufacture microneedle structures.

The hyaluronidase contents in Table 1 represent concentrations of hyaluronidase per one microneedle structure patch. Here, the patch may refer to one sheet of the microneedle structure.

**[Table 1]**

| Classification | Example 1 | Example 2 | Example 3 |
|---|---|---|---|
| Hyaluronidase (IU/patch) | 10 | 300 | 1000 |

### Examples 4 to 8. Manufacturing of Microneedle Structures

Microneedle structures were manufactured in the same manner as in Example 1 except that the ingredients and contents or concentrations listed in Table 2 were applied.

In Table 2, the hyaluronidase contents are concentrations of hyaluronidase per microneedle structure patch, and the unit for ingredients other than hyaluronidase is g.

**[Tabe 2]**

| Classification | | Example 4 | Example 5 | Example 6 | Example 7 | Example 8 |
|---|---|---|---|---|---|---|
| Purified water | | 91.8 | 84.3 | 89.3 | 90.3 | 80.8 |
| Biodegradable polymer | Polyvinyl alcohol | - | - | - | 4 | 4 |
| | Poloxamer | - | 10 | - | - | - |
| | Carbpxymethyl cellulose | 4.5 | 2 | 3 | 2.5 | 2 |
| | Gelatin | - | - | 4 | - | - |
| | Polyvinyl pyrrolidone | - | - | - | - | 10 |
| Saccharide | Mannitol | 2 | 2 | 2 | - | - |
| | Trehalos | - | - | - | 1.5 | 1.5 |
| Hyaluronidase (IU/patch) | | 1000 | 1000 | 1000 | 1000 | 1000 |
| Drug | PN | 1.7 | 1.7 | 1.7 | 1.7 | 1.7 |

### Comparative Example 1. Manufacturing of Microneedle Structure

Microneedle structure was manufactured in the same manner as in Example 1 except that hyaluronidase was not contained.

### Experimental Example 1. Evaluation of Transdermal Absorption of Drug

The experiment as follows was conducted to determine the extent of drug transdermal absorption according to the hyaluronidase content.

A magnetic bar was placed in the cell, and 5 mL of pH 7.4 PBS was injected through the sampling zone, and then the cell was stirred at 600 rpm and heated to 32±1°C. When the temperature was sufficiently warmed, the micropig skin with the microneedle structure attached to the top of the receptor was placed in that order, followed by the gasket, and the upper part of the inserted Franz Cell was fixed so as not to move. As time passes, the drug from the microneedle structure penetrates the skin and mixes with pH 7.4 PBS so that the drug-penetrated solution was sampled with a syringe at a desired time point, and then the cell was filled with pH 7.4 PBS in an amount equal to the sampled solution volume with a syringe, and this was repeated at time points (0.5, 1, 2, 4, 6, 8 h). Here, the above time points are shown as 1 for 0.5h, 2 for 1h, 3 for 2h, 4 for 4h, 5 for 6h, and 6 for 8h. Samples collected at each time point were subjected to confirmation test using a UV/VIS Spectrophotometer (JASCO, V-730) to evaluate the content of the drug that had penetrated the skin through the microneedle structure, 1.5 mL of the sample was placed in a quartz cell and the absorbance was measured at 260 nm using a spectrophotometer. The results are shown in FIGS. 2, 3A and 3B.

Referring to FIGS. 2, 3A, and 3B, it could be confirmed that the drug release amount over time was shown to be somewhat faster in Examples 1 to 3 containing hyaluronidase than in Comparative Example 1, which did not contain hyaluronidase. Comparing absorbance after the passage of 8 hours, it could be confirmed that Example 2, which contained 300 IU of hyaluronidase per sheet, showed a difference of approximately 2.5, and Example 3, which contained 1,000 IU of hyaluronidase per sheet, showed a difference of approximately 3.5, compared to Comparative Example 1, which did not contain hyaluronidase.

Therefore, it could be seen that the drug release rate can be adjusted through the amount of hyaluronidase, or the drug can be delivered to the target site in an appropriate amount as needed.

### Experimental Example 2. Strength Evaluation of Microneedle Structures

Examples 4 to 6 were taken to a size of 10 mm x 10 mm x 10 mm to measure the strengths according to the KSL 1601 test method, and the results are shown in Table 3.

The instrument used was an Instron 5582 from Instron.

**[Table 3]**

| Classification | Force(N) | | | | |
|---|---|---|---|---|---|
| | 1 | 2 | 3 | Average | Criterion |
| Example 4 | 0.34992 | 0.13243 | 0.09545 | 0.192600 | |
| Example 5 | 0.59086 | 0.30034 | 0.65175 | 0.514317 | ≥0.05N |
| Example 6 | 0.28469 | 0.17751 | 0.10872 | 0.190307 | |

Referring to Table 3, average strength values of Examples 4 to 6 were all confirmed to be the reference value of 0.05 N or more. Therefore, it could be seen that the microneedles could reach the dermal layer while not forming wounds in the skin without changes in their shapes.

While exemplary embodiments of the present disclosure have been described in detail above, the scope of rights of the present disclosure is not limited thereto, and various modifications and improvements made by those skilled in the art utilizing the basic concepts of the present disclosure defined in the following claims also fall within the scope of rights of the present disclosure.

Unless otherwise defined, all technical terms used in the present disclosure have been used as the same meaning as commonly understood by those skilled in the art in the relevant fields of the present disclosure. The contents of all publications incorporated in the present specification by reference are incorporated in the present disclosure by reference.

### Description of Reference Numerals

- 100:: Microneedle Structure
- 10:: Substrate
- 20:: Microneedle

## Claims

1. A microneedle structure composition for drug delivery comprising:
a drug; and
a biodegradable composition,
wherein the biodegradable composition comprises hyaluronidase.

2. The microneedle structure composition for drug delivery of claim 1, wherein hyaluronidase is contained in an amount of 10 to 1,500 IU per sheet.

3. The microneedle structure composition for drug delivery of claim 1, wherein the biodegradable composition comprises one or more selected from a biodegradable polymer, a saccharide, and an additive.

4. The microneedle structure composition for drug delivery of claim 3, wherein the biodegradable polymers are contained in an amount of 50 to 99 parts by weight per 100 parts by weight of the biodegradable composition.

5. A microneedle structure for drug delivery comprising:
a substrate; and
one or more microneedles disposed on the substrate,
wherein the microneedles contain a drug and a biodegradable composition, and the biodegradable composition comprises hyaluronidase.

6. The microneedle structure for drug delivery of claim 5, wherein the microneedles increase the transdermal absorption rate of the drug compared to microneedles that do not contain hyaluronidase.

7. The microneedle structure for drug delivery of claim 5, wherein the microneedles have a height of 100 to 1,500 µm.

8. The microneedle structure for drug delivery of claim 5, wherein the microneedle structure for drug delivery has a strength of 0.05 N or more.
